# EUROPEAN PATENT SPECIFICATION

(11) **EP 3 228 355 B1**
(45) Date of publication and mention of the grant of the patent: **21.04.2021**
(21) Application number: 16193176.1
(22) Date of filing: 12.10.2009
(51) Int. Cl.: A61N 1/378, A61F 2/02, A61N 1/05, A61N 1/372, G10L 15/05, A61B 5/00, A61B 5/07, G10L 15/00, H04B 13/00, G10L 15/06

(54) **ENERGY FEEDBACK CAPACITIVE COUPLING DATA SYSTEM**
KAPAZITIVES ENERGIERÜCKKOPPLUNGSDATENSYSTEM
SYSTÈME DE DONNÉES DE RÉCUPÉRATION D'ÉNERGIE DE COUPLAGE CAPACITIF

(30) Priority: 10.10.2008 SE 0802163; 23.07.2009 US 227822 P
(43) Date of publication of application: 11.10.2017
(62) Divisional of application: 09819520.9
(73) Proprietor: Implantica Patent Ltd., 223 70 Lund (SE)
(72) Inventor: FORSELL, Peter, 6341 Baar (CH)

(56) References cited:
- WO-A1-01/56653
- WO-A1-2009/051541
- US-A1- 2003 187 490
- US-A1- 2006 271 128
- US-A1- 2007 156 204

## Description

### TECHNICAL FIELD

The present invention relates generally to transferring information between a device placed outside a person's or patient's body and another device placed inside the person's or patient's body.

### BACKGROUND

A medical device, designed to be subcutaneously implanted, may include e.g. electronic circuits and/or some other electric device and hence it needs, like any other similar device, electric power for its operation. Examples of such medical devices include e.g. electrical and mechanical stimulators, motors, pumps, valves and constriction devices that can support, stimulate or control various body functions of the person in which the medical device is implanted.

Electric power to a medical device implanted in a person's body can as conventional be supplied from an implanted electrical energy storage such as one or more electrochemical cells. Electric power to devices located inside a person's body can also be supplied from outside the body using wireless energy transfer, for example supplying electric power using electrical induction. An external device used in such case for transmitting electric power can be called an electric power transmitter. The wirelessly supplied power can be used to directly operate an implanted medical device or to charge an implanted electrical energy storage.

A device suited to be implanted can also require electrical signals in order to generally control its operation and its functions such as, in a simple case, to start or stop the operation of the device. For more complicated devices or devices having more complicated functions, the device could also be required to provide feedback, i.e. signals representing the current state of the device or its function or e.g. some value sensed by the implanted device. Such signals can be exchanged with an external device, e.g. a controller. Then, the signals can also be wirelessly transferred.

Wireless supply of power and wireless exchange of signals have for a medical implant the obvious advantage that no electrical line extending through the skin of the person having the implant is required.

Wireless communication of signals of course also requires electrical power. This is of special importance considering implanted devices and the communication should be designed to require as small electrical power and energy as possible from the implants WO2009051541 discloses a supply apparatus for wirelessly supplying power to medical implant, which has a feedback communication system that sends feedback information from the receiver the external energizer by using patient's body as electrical signal line.

### SUMMARY

In the system described herein for communication of information between a medical implant implanted in a patient's body and an external communication unit provided outside the patient's body, generally the external communication unit has a part adapted to be in contact with or be placed in a close vicinity of the patient's body when in use and the medical implant comprises an internal communication unit. The external communication unit and the internal communication unit communicates with each other using a communication path, a part of which uses or includes the patient's body for the communication of information.

The communicated information can be digital in the common way. It can then be represented as transitions of a signal, such as transitions between states or levels of a signal. For binary information two states or levels are used. The communicated information can also be generally represented as variations of the derivative of a signal, the derivative taken in regard of time. For a binary signal thus a zero is represented by a transition of a first kind and a one is represented by a transition of a second, different kind. Such a method of communicating information requires that in receiving and decoding the signal, a clock signal for the times when the transitions occur, such as for the start time of the transitions, is available.

In representing the communicated information, the states of a signal can e.g. include different frequencies of the signal, the derivate then taken of the frequency in regard of time. Thus, e.g. dual frequency communication can be used. Furthermore, in representing the communicated information, e.g. Manchester encoding can be used, i.e. the information can be coded according to the Manchester system.

In particular, the system is based on the realization that by using the patient's body as a communication medium and measuring the electric potential in different places, communication between a medical implant and a communication unit outside the patient's body can be established with a minimum of electric current flowing through the body. In particular, a portion of the patient's body is used as part of a capacitor. Generally then, the internal communication unit comprises a communication receiver, transmitter or transceiver that includes one part of a capacitive energy storage. The communicating of information using the capacitive coupling includes that an electrical current is injected into or is drawn from the capacitive energy storage:
In the system, the information can e.g. be represented as variations of the derivative of the voltage over the capacitive energy storage, i.e. as transitions in the voltage level.

Further embodiments are defined by the dependent claims.

### BRIEF DESCRIPTION OF THE DRAWINGS

While the novel features of the invention are set forth with particularly in the appended claims, a complete understanding of the invention, both as to organization and content, and of the above and other features thereof may be gained from and the invention will be better appreciated from a consideration of the following detailed description of non-limiting embodiments presented hereinbelow with reference to the accompanying drawings, in which:
- Fig. 1 is a schematic of a system for transferring information,
- Fig. 2 is an overview circuit diagram of driver circuits in the system of Fig. 1,
- Fig. 3 is a schematic illustrating two ways of locating the system of Fig. 1,
- Fig. 4a is a front view of internal components of the system of Fig. 1 in a common housing,
- Fig. 4b is a front view similar to Fig. 4a of an alternative embodiment,
- Fig. 4c is a picture of a possible design of dual capacitor plates,
- Fig. 5a is a diagram of pulses generated by a microcontroller according to a Manchester scheme,
- Fig. 5b is a diagram of signals detected in a first stage,
- Fig. 5c is a diagram of signals detected by a comparator,
- Fig. 6 is a circuit diagram of a transceiver that uses Manchester coded and is included in the system of Fig. 1, and
- Fig. 7 is similar to Fig. 6 but of a transceiver that uses amplitude modulation.

### DETAILED DESCRIPTION

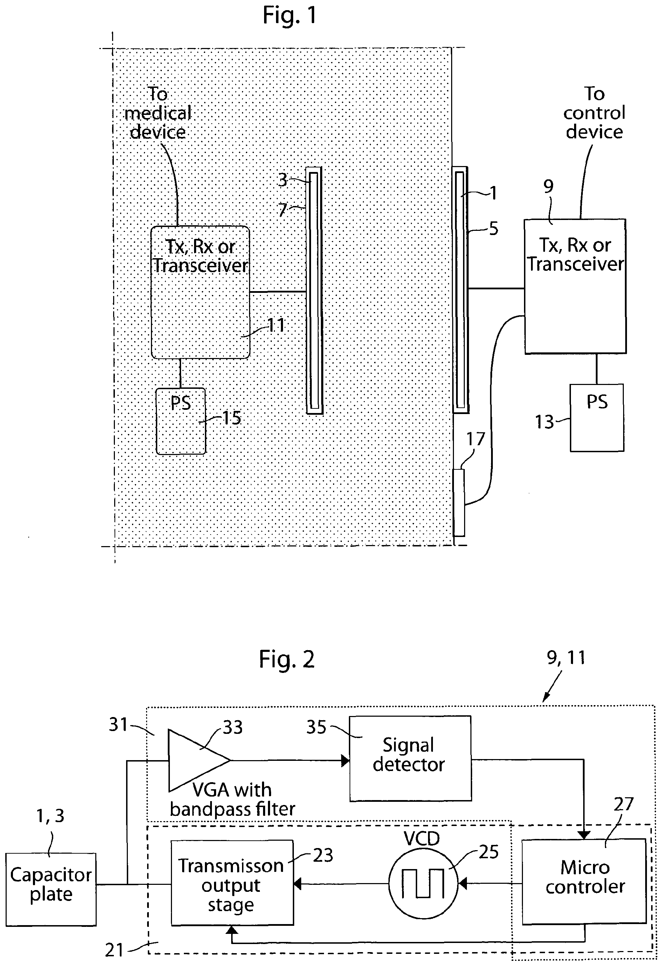

Fig. 1 is a schematic of a system for transferring information to and/or from an implanted device, not shown, illustrating a principle of capacitive signal transmission. The system includes an electric capacitor formed by two capacitor plates 1, 3 and intermediate regions of material, in particular a region of the patient's body. The first capacitor plate 1 is located outside the body of the person in which the device is implanted. The plate can be applied to the skin of the person's body or be placed in a close vicinity thereof. The second capacitor plate 3 is located inside the person's body and is thus also an implant. Since the body tissues are electrically conducting, the capacitor plates 1, 3 must be electrically insulated in order to form the intended capacitor. Hence, each of the capacitor plates is embedded in an electrical insulator 5, 7, the insulator e.g. forming a thin layer totally surrounding the respective plate that is made from an electrically conducting material, e.g. a well conducting material such as copper. The electrical resistance between the capacitor plates should be as high as possible, e.g. at least 1 MΩ.

The capacitor plates 1, 3 are electrically connected to driver circuits 9, 11, respectively, which basically can include transmitter, receiver or transceiver circuits. The driver circuits 11 for the internal capacitor plate 3 are thus also implanted and are electrically connected to the implanted device or a control device therefor, not shown. The external driver circuits 9 are connected to a control device, not shown. The driver circuits are powered by power supplies 13, 15, the internal one also being implanted. The driver circuits may require a common electrical ground potential which can make the transfer of information more secure. Thus, the driver circuits of the internal communication unit can include or be connected to an electrically conducting portion, such as a plate or electrode, suited to be in electrical contact and connection with internal body tissues. In another example, the housing of the internal driver circuits 11 is at least partly electrically conducting, so that the electrically conducting area thus is in electrical connection with the body tissues. For the same purpose, the external driver circuits 9 can be electrically connected to an electrically conducting portion, such as an electrically conducting plate or electrode 17, that is attached to and electrically connected to the skin of the person's body in the same way as electrodes e.g. for cardiography.

The capacitor formed by the capacitor plates 1, 3 is part of an electric circuit connection between the driver circuits 9, 11 and electric signals can be transmitted over this circuit connection. By selecting the dimensions of the plates and their location in relation to each other the capacitor can be given a capacitance suited for the signal transfer. Hence, the plates can be made to have as large a surface area as is possible for an implant, e.g. in the range of 2 - 8 cm², and be configured in a suitable way. Of course, they may be rectangular or square plates but they may also e.g. have an elongated round shape or a circular shape. In particular, the internal capacitor plate 3 can be given a suitable shape, making it suitable to be implanted. Thus, it may e.g. have perforations or through-holes, not shown, allowing it to be securely attached in body tissues.

The driver circuits can be designed as is schematically illustrated in Fig. 2. The capacitor plate 1, 3 is thus connected to a transmission stage 21 that includes a transmission output stage 23, receiving an input signal a wave or alternating electric signal from an oscillator circuit 25, e.g. a voltage controlled oscillator (VCO) as illustrated, the oscillator circuit and the transmission output stage both being controlled by a microcontroller 27 such as for commanding a special wave form and for modulating it, respectively. The capacitor plate is also connected to a receiving stage 31, that includes an amplifier 33 also working as a bandpass filter. The amplifier provides its output signal to a signal detector 35 which delivers the detected information signal to the microcontroller 27. The driver circuits for the external and internal capacitor plates can include either of the transmission and receiving stages 21, 31 or both. The microcontroller can be the type PIC16F818 and it thus controls the transmission and receiving stage. For the receive mode, the microcontroller converts the signal level received from the signal detector 35, then using an ADC such the on-chip 8-bit ADC built into the PIC16F818.

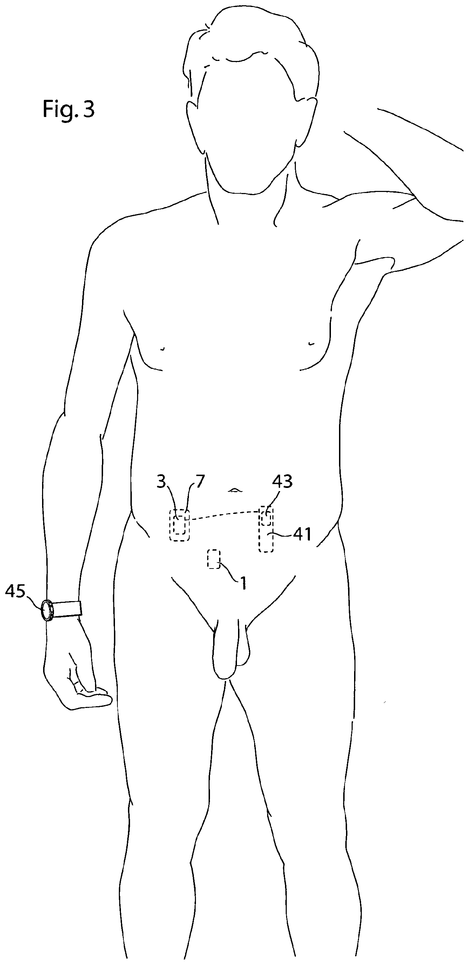

The system can be arranged on a person's body such as illustrated in Fig. 3. The capacitor plates 1, 3 can e.g. be arranged at the person's waist region. The internal capacitor plate 3 is seen to be surrounded by the electrical insulator 7, this for instance comprising two electrically insulated sheets between the electrically conducting plate is placed, the sheets being welded to each other at their margin region to provide a hermetic enclosure. In particular, the internal capacitor plate can be integrated with its driver circuits and power supply in one enclosure, the driver circuits connected to an implanted device 41 having its own power supply 43, e.g. an electrochemical cell. Alternatively, the driver circuits can be integrated with control circuits provided for the implanted device and then the power supply 43 also supplies power to the driver circuits.

In a special embodiment, the external capacitor plate 1 and its driver circuits 9 and power supply 13 can be integrated in a wristwatch 45. The internal capacitor plate 3, not seen in Fig. 3 for this case, may be located directly under the person's skin region.

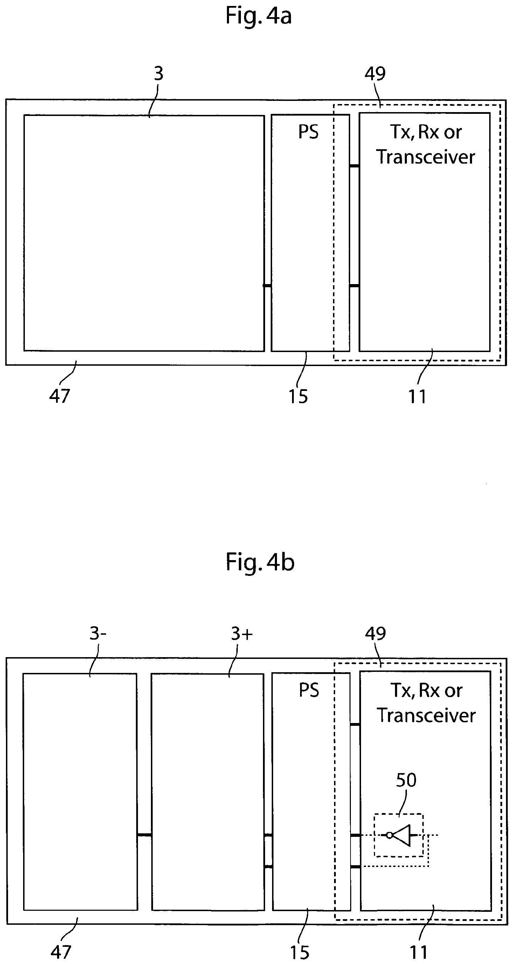

In Fig. 1 the driver circuits 11, the power supply 15 and the internal capacitor plate 3 are seen to be separate units, only connected by electrical cables. They can also be integrated as one single unit, placed at the sides of each other inside a common enclosure or housing 47, see Fig. 4a, which can be made from an electrically insulating material, forming the necessary electrically insulation of the capacitor plate.

The communication channel or path having a capacitive coupling as described above should have a constant impedance which is as small as possible in order to ensure that the communication signals are appropriately transferred. However, the capacitance of the capacitor used having one capacitor plate implanted in a patient's body is not constant due to the facts that the plates can move in relation to each other and that body functions in the tissues located between the capacitor plates can change. The frequency used for the communication is substantially constant if e.g. a carrier signal which is modulated is used or pulses of a definite frequency is used. Also, the frequency has to be as large as possible to make the impedance small.

In order to improve the total capacitive coupling between the capacitor plates 1, 3 they can be divided to each include a first plate and a second plate, see Fig. 4b. For transmitting a signal through the capacitor, the two plates on the sending side can be then provided with signals which are the inverse of each other. Thus, e.g. the first plate can be provided with the direct signal and be denoted 3+ and the second plate can be provided with the inverted signal and then be denoted 3-. The inversion of signals can be easily achieved by arranging an inverter circuit such as that indicated at 50. For receiving an inverter circuit, not shown, having the opposite direction can be used. The external capacitor plate 1 must be configured in a similar and corresponding way, having one portion, not shown, for the direct signal and one portion, not shown, for the inverted signal.

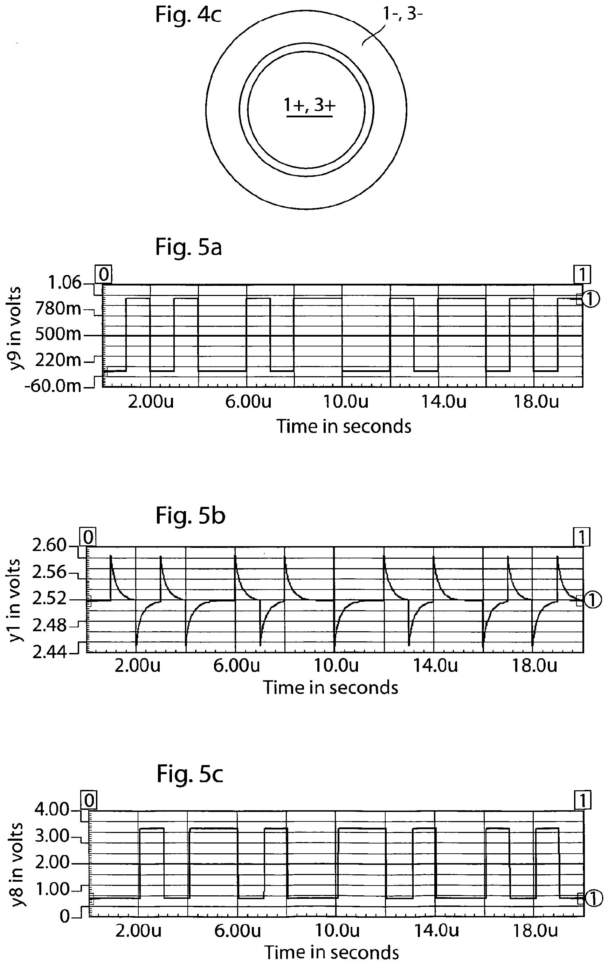

The dual capacitor plates used in this case can for ease of positioning be configured as concentric circular fields as seen Fig. 4c, at least one of which is annular. One capacitor portion 1+, 3+ can e.g. be a central circular field that is surrounded by an annular circular field 1-, 3-.

Various ways of communicating signals over the communication path involving a capacitive coupling can be conceived, considering the above mentioned conditions of the signal transmission, and possible methods will now be described.

In the simplest case, the signals used in the communication between the external and internal devices can e.g. be electric pulses, e.g. substantially rectangular pulses. However, since the communication of information in most case must be made with a high degree of security, a suitable coding of the information could be used. Hence, e.g. Manchester coding can be used.

Manchester encoding is a special case of binary phase shift keying where each bit of data is signified by at least one transition. The encoding is therefore self-clocking which makes accurate synchronization of the data stream possible. For example, a "1" can be represented by a transition from a high to a low level and a "0" can be represented by a transition from a low to a high electrical level. This means that in the derivative of the electrical signal in regard of time, there are variations so that a "1" can be seen as a negative pulse and a "0" as a positive pulse. In the electrical signal there are also transitions between the two levels that do not represent any information but are necessary in order that the transitions representing information can be arranged in the electrical, such extra transitions thus inserted when sending two equal consecutive bits of information.

Generally, the digital information to be communicated can be represented as transitions of a signal, such as transitions between states or levels of a signal, these states or levels being for example different frequencies, different voltage levels or different levels of the amplitude of a carrier wave. For binary information only two states or levels are required. For a binary signal comprising two symbols thus a "0" is represented by a transition of a first kind such as from a first state to a second state and a "1" is represented by a transition of a second, different kind such as from a second state to a first state. In a signal containing information that is conveyed in the system considered here, however, there are always consecutive sequences of the same symbol, such as of "0:s" and "1:s". The first symbol in such a sequence is then represented by the respective transition but for the following symbols in the sequence this transition can obviously not be used. Thus, in one case, for the following symbols no transition at all is used. Since the transitions are sent in a fixed rate, these following symbols can still be decoded. Thus, such a method requires that in receiving and decoding the signal, a clock signal for the times when the transitions are supposed to occur, such as for the start times of the transitions, is available. A clock signal can be obtained in the receiving side by sending, from the transmitting side, initially or at suitably repeated times, a sequence of transitions in the fixed rate, such a sequence then represented e.g. by the symbol sequence "10101010...".

Each of the symbols or bits of the communicated information can thus be generally represented as a variation of the derivative of a signal, the derivative taken in regard of time. Such a variation can then be e.g. positive pulse or a negative pulse.

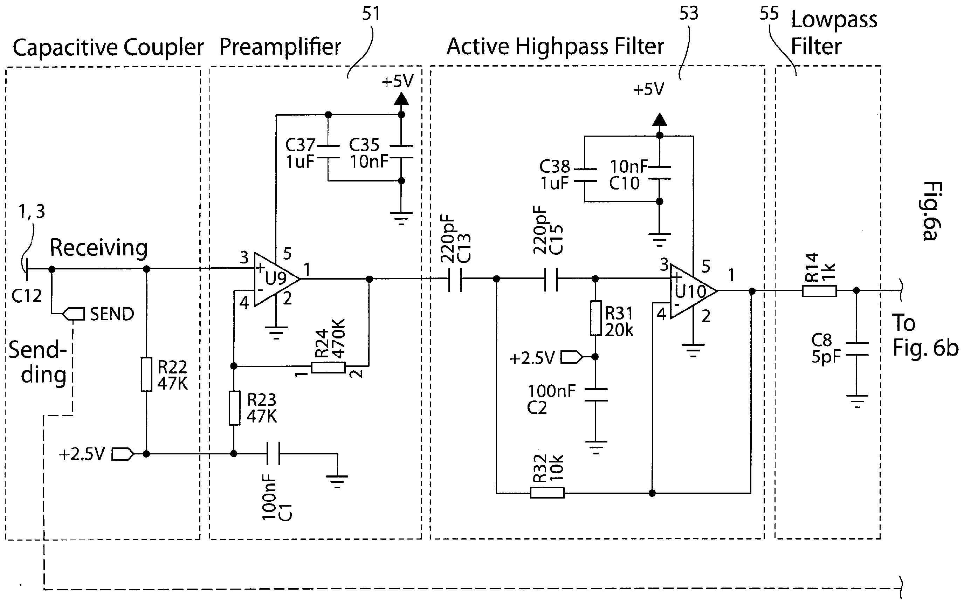

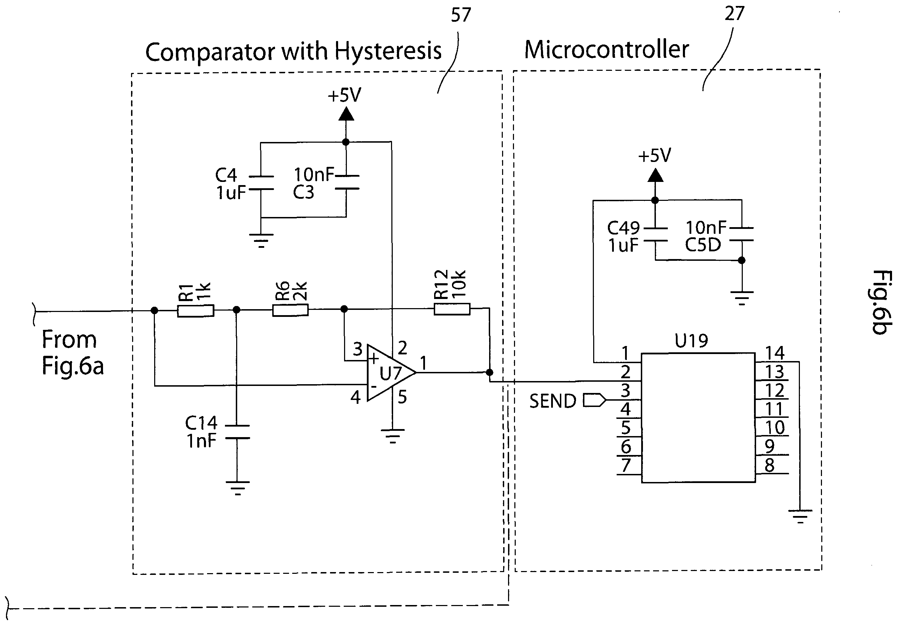

For the cases of simple pulse transmission, the transmitter output stage 23 and the oscillator 25 illustrated in the circuit diagram of Fig. 2 may not be required since the pulses can be generated directly in the microprocessor 27 and provided to the respective capacitor plate 1, 3. A typical Manchester encoded signal generated by a microcontroller is illustrated in the diagram of Fig. 5a. Fig. 6 is a circuit diagram of driver circuits 9, 11 comprising a transceiver that can be used in this case.

For receiving, the transmitted signal is picked up by the capacitor plate 1, 3. The DC level of the signal is by the resistor R22 pulled to 2.5V which is equal to VCC/2. The received signal is provided to a preamplifier stage 51 including an amplifier U9 before it is passed to filter stages. The amplifier has a high input impedance and a low bias current. The signal is then provided to a highpass filter stage 53 that is configured as a second order active high pass filter including an amplifier U10 as its active element. This filter stage removes low frequency interfering signals and noise. Then, the signal is passed to a lowpass filter stage 55 being a passive filter of RC-type, comprising a resistor R41 and a capacitor C8 to remove high frequency noise.

The signal is then provided to a signal detector stage that here is designed as a comparator 57 stage having hysteresis. Thus, the received and filtered signal is fed to the inverting input of a comparator U7. The same signal is also first even more low pass filtered in a passive RC-filter including R41 and C14 and then fed to the non-inverting input of the comparator via a resistor R6. The resistor R6 and the feedback resistor R12 form the hysteresis feedback. The comparator U7 has hysteresis in order to output a square wave in Manchester code even if the signal drops down below the DC level. An example of a received and filter signal can be seen in Fig. 5b and the output from the comparator U7 in Fig. 5c.

The microcontroller U19 is used to decode the received Manchester stream into useful data. This is achieved by measuring the time between rising and falling edges. When a reception is initialized, the microcontroller receives a preamble consisting of the repeated pattern "10101010". Since the only transitions that occur in that pattern are the bit transitions the preamble can be used to synchronize the data, i.e. to form a clock signal. When synchronization has been accomplished, the microcontroller can begin to translate the Manchester stream into useful data.

Another method mentioned above is to use amplitude modulation to transfer data. For instance, a carrier frequency can be on/off-modulated to output bursts of the electrical signal.

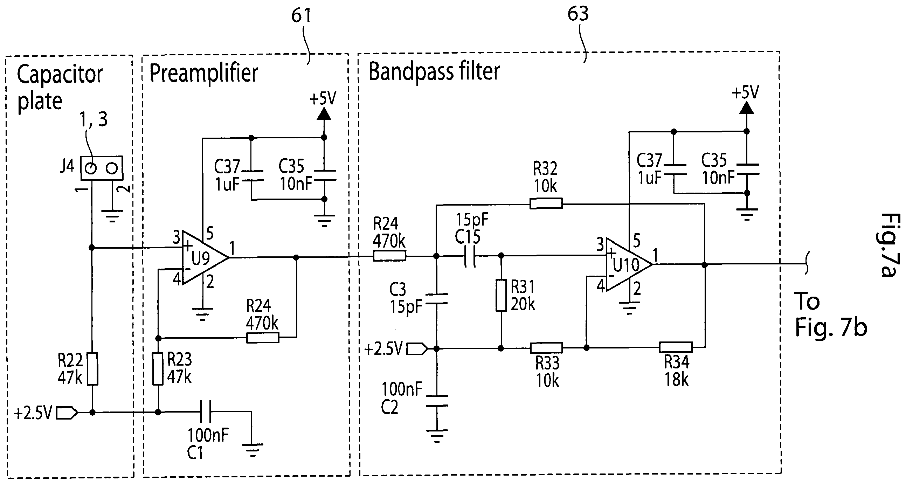

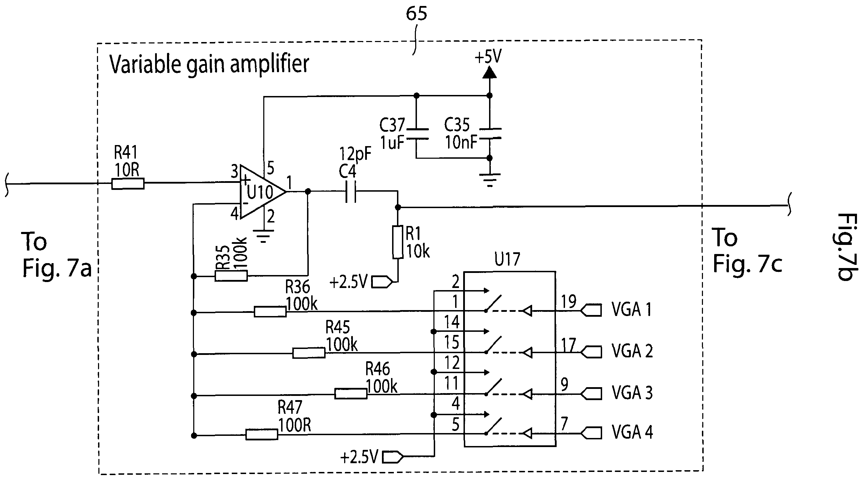

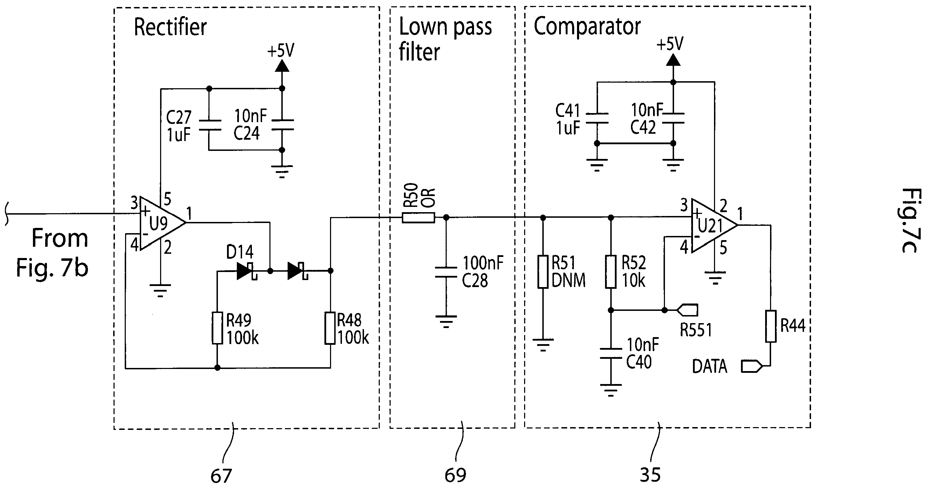

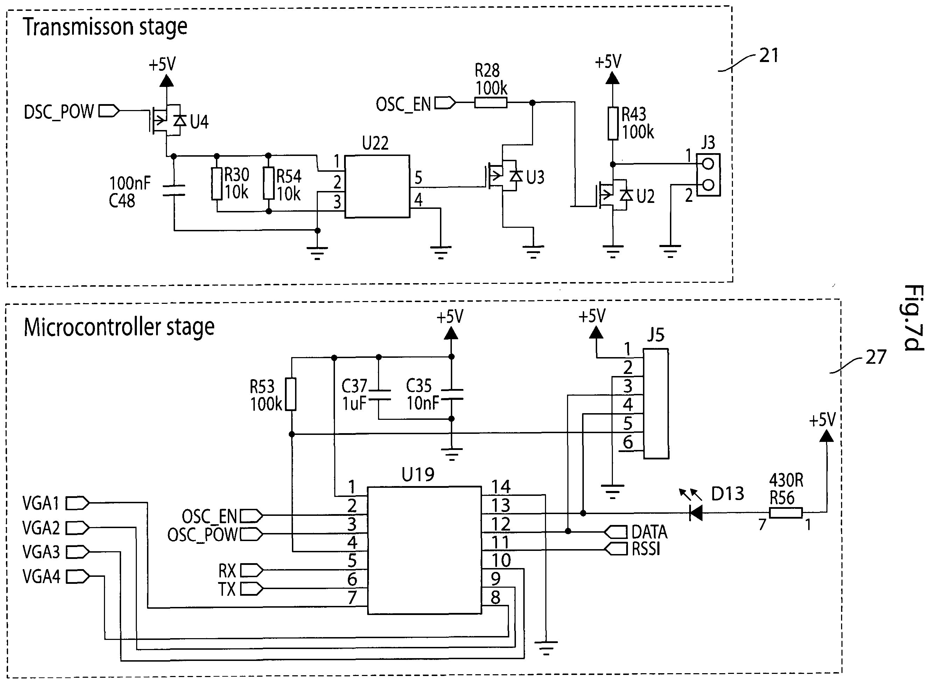

For this method driver circuits like those illustrated in Fig. 7 may for instance be used. The transmission stage 21 has a signal generator U22, that can be enabled by a signal "OSC_POW" from the microcontroller U19 in the microcontroller stage, the signal opening a transistor U4. The signal generator U22 outputs a oscillatory signal having a frequency of about 1.4 MHz that is set by the resistors R30 and R54. The output from the signal generator U22 is fed to the gate of another transistor U3. Another signal "OSC_EN" from the microcontroller is used to modulate the amplitude of the signal by being provided the gate of a transistor U2. The transistor U2 and resistor R43 are provided to make it possible to transmit a voltage higher than 5V.

For receiving information, the transmitted signal is picked up by the capacitor plate connected to J4. The DC level of the received signal is by the resistor 22 pulled to 2.5V which is equal to VCC/2. The received is provided to a preamplifier stage 61 including an amplifier U9 having a high input impedance and a low bias current. The amplified signal is passed to a bandpass filter 63. The bandpass filter is a second order active band pass filter including an amplifier U10 as its active element. The filtered signal is provided to a variable gain amplifier 65 including a non-inverting amplifier U13. A resistor connects the inverting input of the non-inverting amplifier to a reference voltage that can be chosen by setting an analog switch U17. The gain of the variable gain amplifier 65 can therefore be set by the microcontroller 27 by control signals "VGA1-4". After having passed the variable gain amplifier, the signal is half-wave rectified in a rectifier stage 67 including an amplifier U18 having two diodes D14 connected in its feedback loop. The rectified signal is by a passive low pass RC-filter 69 including a resistor R50 and a capacitor C28 to output a rectangular wave. The rectangular wave is high when the amplitude of the received signal is high or on and it is low when the amplitude is off or zero. Finally, the wanted signal is detected in a signal detector or comparator stage 35 by being provided to the non-inverting input of a comparator U21. The signal is also simultaneously low pass filtered by the RC-filter arranged by the resistor R52 and the capacitor C40 to provide an averaged signal to the inverting input. The signal "DATA" output from the comparator U21 is fed to the microcontroller 27 to decode the received data.

For the data reception to work properly in this case it may be important that the transmitted signal is balanced in the meaning that it is on and off for the same amount of time. The data can for that reason, also in this case, be encoded using Manchester code as described above.

A development of the simple amplitude modulation method using a carried that is switched on and off is the method called frequency shift keying (FSK). This modulation scheme represents a digital '0' with a first frequency and a '1' with a second, different frequency where these frequencies can be selected to be as large as possible. If possible, also rectangular waves can be used instead of sine waves to get a better transmission through the capacitive link.

In demodulating, in this case a received frequency is transformed into a '0' or '1'. This can be done using a phase locked loop (PLL), in particular a digital phase locked loop (DPLL). Such a digital demodulating circuit comprises a pfd or phase detector, a loop filter, a VCO counter and a decider. The phase detector looks on the incoming signal and compares it to the generated signal in the VCO counter. If any of the signals goes high before the other, this information is sent to the loop filter. The loop filter gets the information about which signal goes high first and translates this to a control signal for the VCO counter. This signal is the preset for the counter inside VCO counter. The VCO counter is a counter that always counts down and has a load and preset inputs. These inputs are controlled by the loop filter. The decider is a unit or circuit which creates the data signal. This is done by looking at the preset signals and, depending on the value, choosing between a '0' and a '1'.

While specific embodiments of the invention have been illustrated and described herein, it is realized that numerous other embodiments may be envisaged and that numerous additional advantages, modifications and changes will readily occur to those skilled in the art without departing from the scope of the invention. The invention is set out in the appended claims.

## Claims

1. A system for communication of information, comprising:
a medical implant (41) for implantation in a patient's body; and
an external communication unit (45) adapted to be arranged outside the patient's body and to be placed in contact with, or in close vicinity of, the patient's body when in use;
wherein:
the information is communicated between the medical implant, when implanted in the patient's body, and the external communication unit;
the medical implant (41) is connected to or comprises an internal communication unit (47);
the external communication unit comprises a first dual capacitor plate (1) formed of two plates;
the internal communication unit comprises a second dual capacitor plate (3) formed of two plates;
the external communication unit (45) and the internal communication unit (47) communicate with each using a capacitive coupling between the first dual capacitor plate and the second dual capacitor plate, using a part of the patient's body as a communication path;
the communicated information is digitally represented as transitions of a signal, wherein the transitions of the signal are transitions between states or levels of a signal, or variations of the derivative of a signal, wherein the transitions occur at a fixed rate, wherein said fixed rate is obtained by a receiving one of the external and the internal communication unit by transmitting, from a transmitting one of the external and the internal communication unit, a sequence of transitions at the fixed rate.

2. The system according to claim 1, wherein the sequence of transitions at the fixed rate is transmitted initially when communicating the information between the internal and the external communication unit.

3. The system according to any one of claims 1-2, wherein the sequence of transitions at the fixed rate is transmitted at repeated times.

4. The system according to any one of claims 1-3, wherein the sequence of transitions at the fixed rate is represented by the symbol sequence "10101010...".

5. The system according to any one of claims 1-4, wherein the internal communication unit (47) comprises a communication transceiver (11) comprising a part of a capacitive energy storage.

6. The system according to claim 5, wherein the communicating of information using the capacitive coupling includes that an electrical current is injected into or is drawn from the capacitive energy storage.

7. The system according to claim 6, wherein the external communication unit comprises a communication transceiver (9) comprising a part of a capacitive energy storage, wherein the communicating of information using the capacitive coupling includes that an electrical current is injected into or is drawn from the capacitive energy storage, and wherein the communicated information is represented by variations of a derivative of the voltage over the capacitive energy storage.

8. The system according to any one of claims 1-7, wherein the two plates of the first dual capacitor plate and the second dual capacitor plate, respectively, have a circular symmetry and are concentrically arranged relative each other.

9. The system according to any one of the preceding claims, wherein, in representing the communicated information, Manchester encoding is used.

10. The system according to any one of the preceding claims, wherein the two plates each of the first and second dual capacitor plates are provided with signals that are the inverse of each other.

11. The system according to any one of the preceding claims, wherein each of the first and second capacitor plate is electrically connected to a respective driver circuit, wherein the driver circuits of the first and second dual capacitor have a common electrical ground potential.

12. The system according to claim 11, wherein the driver circuit of the internal communication unit includes or is connected to an electrically conducting portion to be in electrical contact with internal body tissue when implanted, and wherein the driver circuit of the external communication unit includes or is connected to an electrically conducting portion arranged to be electrically connected to the skin of the person's body.

13. The system according to claim 12, wherein the electrically conducting portion of the driver circuit of the internal communication unit is part of a housing for said driver circuit.

14. The system according to claim 13, wherein the electrical resistance of the communication path is at least 1 MΩ.

15. The system according to claim 1, wherein the state or levels of the signal include at least one of different frequencies of the signal, different phases of the signal, and different pulse characteristics such as different voltage levels and different levels of amplitude of the carrier wave.

## Patentansprüche

1. System zur Kommunikation von Informationen, umfassend:
ein medizinisches Implantat (41) zur Implantierung in den Körper eines Patienten; und
eine externe Kommunikationseinheit (45), die vorgesehen ist, um außerhalb des Körpers des Patienten angeordnet und bei Gebrauch in Kontakt mit oder in enger Nähe des Körpers des Patienten zu sein;
wobei:
die Informationen zwischen dem medizinischen Implantat, wenn es in den Körper des Patienten implantiert ist, und der externen Kommunikationseinheit kommuniziert werden;
das medizinische Implantat (41) mit einer internen Kommunikationseinheit (47) verbunden ist oder diese umfasst;
die externe Kommunikationseinheit eine erste Doppelkondensatorplatte (1) umfasst, die aus zwei Platten gebildet ist;
die interne Kommunikationseinheit eine zweite Doppelkondensatorplatte (3) umfasst, die aus zwei Platten gebildet ist;
die externe Kommunikationseinheit (45) und die interne Kommunikationseinheit (47) miteinander unter Verwendung einer kapazitiven Kopplung zwischen der ersten Doppelkondensatorplatte und der zweiten Doppelkondensatorplatte kommunizieren, wobei ein Teil des Körpers des Patienten als Kommunikationspfad verwendet wird;
die kommunizierten Informationen digital als Übergänge eines Signals repräsentiert werden, wobei die Übergänge des Signals Übergänge zwischen Zuständen oder Niveaus eines Signals sind, oder Variationen der Ableitung eines Signals sind, wobei die Übergänge mit einer Fixrate stattfinden, wobei die Fixrate durch eine empfangende von der externen und der internen Kommunikationseinheit erhalten wird, indem von einer sendenden von der externen und der internen Kommunikationseinheit eine Sequenz von Übergängen mit der Fixrate gesendet wird.

2. System nach Anspruch 1, wobei die Sequenz der Übergänge mit der Fixrate am Anfang gesendet wird, wenn die Informationen zwischen der internen und der externen Kommunikationseinheit kommuniziert werden.

3. System nach einem der Ansprüche 1 bis 2, wobei die Sequenz der Übergänge mit der Fixrate wiederholt gesendet wird.

4. System nach einem der Ansprüche 1 bis 3, wobei die Sequenz der Übergänge mit der Fixrate durch die Symbolsequenz "10101010..." repräsentiert wird.

5. System nach einem der Ansprüche 1 bis 4, wobei die interne Kommunikationseinheit (47) einen Kommunikationssendeempfänger (11) umfasst, der ein Teil eines kapazitiven Energiespeichers umfasst.

6. System nach Anspruch 5, wobei das Kommunizieren von Informationen unter Verwendung der kapazitiven Kopplung einschließt, dass ein elektrischer Strom in den kapazitiven Speicher einfließen gelassen oder aus diesem abgezogen wird.

7. System nach Anspruch 6, wobei die externe Kommunikationseinheit einen Kommunikationssendeempfänger (9) umfasst, der einen Teil eines kapazitiven Energiespeichers umfasst, wobei das Kommunizieren von Informationen unter Verwendung der kapazitiven Kopplung einschließt, dass ein elektrischer Strom in den kapazitiven Energiespeicher einfließen gelassen oder aus diesem abgezogen wird, und wobei die kommunizierten Informationen durch Variationen einer Ableitung der Spannung über dem kapazitiven Energiespeicher repräsentiert werden.

8. System nach einem der Ansprüche 1 bis 7, wobei die beiden Platten der ersten Doppelkondensatorplatte beziehungsweise der zweiten Doppelkondensatorplatte eine kreisförmige Symmetrie haben und relativ zueinander konzentrisch angeordnet sind.

9. System nach einem der vorhergehenden Ansprüche, wobei Manchester-Kodierung verwendet wird, um die kommunizierten Informationen zu repräsentieren.

10. System nach einem der vorhergehenden Ansprüche, wobei den beiden Platten von jeder von der ersten und der zweiten Doppelkondensatorplatte Signale bereitgestellt werden, die invers zueinander sind.

11. System nach einem der vorhergehenden Ansprüche, wobei jede von der ersten und der zweiten Kondensatorplatte elektrisch mit einer jeweiligen Treiberschaltung verbunden ist, wobei die Treiberschaltungen des ersten und des zweiten Doppelkondensators ein gemeinsames elektrisches Erdungspotential haben.

12. System nach Anspruch 11, wobei die Treiberschaltung der internen Kommunikationseinheit einen elektrisch leitfähigen Abschnitt einschließt oder damit verbunden ist, der in elektrischen Kontakt mit internem Körpergewebe kommen soll, wenn er implantiert wird, und wobei die Treiberschaltung der externen Kommunikationseinheit einen elektrisch leitfähigen Abschnitt einschließt oder damit verbunden ist, der vorgesehen ist, um elektrisch mit der Haut des Körpers einer Person verbunden zu werden.

13. System nach Anspruch 12, wobei der elektrisch leitfähige Abschnitt der Treiberschaltung der internen Kommunikationseinheit Teil eines Gehäuses für die Treiberschaltung ist.

14. System nach Anspruch 13, wobei der elektrische Widerstand des Kommunikationspfads mindestens 1 MΩ beträgt.

15. System nach Anspruch 1, wobei die Zustände oder Niveaus des Signals mindestens eins von unterschiedlichen Frequenzen des Signals, unterschiedlichen Phasen des Signals und unterschiedlichen Pulscharakteristika, wie unterschiedlichen Spannungsniveaus und unterschiedlichen Amplitudenniveaus der Trägerwelle, einschließt.

## Revendications

1. Système pour une communication d'informations, comprenant ;
un implant médical (41) pour une implantation dans le corps d'un patient ; et
une unité de communication externe (45) conçue pour être disposée à l'extérieur du corps du patient et pour être placée en contact avec le corps du patient, ou au voisinage étroit de celui-ci, lors de l'utilisation ;
dans lequel :
les informations sont communiquées entre l'implant médical, lorsqu'il est implanté dans le corps du patient, et l'unité de communication externe ;
l'implant médical (41) est connecté à ou comprend une unité de communication interne (47) ;
l'unité de communication externe comprend une première plaque de condensateur double (1) formée de deux plaques ;
l'unité de communication interne comprend une seconde plaque de condensateur double (3) formée de deux plaques ;
l'unité de communication externe (45) et l'unité de communication interne (47) communiquent entre elles à l'aide d'un couplage capacitif entre la première plaque de condensateur double et la seconde plaque de condensateur double, en se servant d'une partie du corps du patient comme trajet de communication ;
les informations communiquées sont représentées numériquement en tant que transitions d'un signal, les transitions du signal étant des transitions entre des états ou des niveaux d'un signal, ou des variations de la dérivée d'un signal, les transitions se produisant à une fréquence fixe, ladite fréquence fixe étant obtenue par une unité réceptrice parmi l'unité de communication externe et interne en émettant, depuis une unité émettrice parmi l'unité de communication externe et interne, une séquence de transitions à la fréquence fixe.

2. Système selon la revendication 1, dans lequel la séquence de transitions à la fréquence fixe est émise initialement lors de la communication des informations entre l'unité de communication interne et externe.

3. Système selon l'une quelconque des revendications 1 et 2, dans lequel la séquence de transitions à la fréquence fixe est émise à des temps répétés.

4. Système selon l'une quelconque des revendications 1 à 3, dans lequel la séquence de transitions à la fréquence fixe est représentée par la séquence de symboles « 10101010... ».

5. Système selon l'une quelconque des revendications 1 à 4, dans lequel l'unité de communication interne (47) comprend un émetteur-récepteur de communication (11) comprenant une partie d'un stockage d'énergie capacitive.

6. Système selon la revendication 5, dans lequel la communication d'informations à l'aide du couplage capacitif inclut qu'un courant électrique soit injecté dans le stockage d'énergie capacitive ou soit consommé depuis celui-ci.

7. Système selon la revendication 6, dans lequel l'unité de communication externe comprend un émetteur-récepteur de communication (9) comprenant une partie d'un stockage d'énergie capacitive, dans lequel la communication d'informations à l'aide du couplage capacitif inclut qu'un courant électrique soit injecté dans le stockage d'énergie capacitive ou soit consommé depuis celui-ci, et dans lequel les informations communiquées sont représentées par des variations d'une dérivée de la tension sur le stockage d'énergie capacitive.

8. Système selon l'une quelconque des revendications 1 à 7, dans lequel les deux plaques de la première plaque de condensateur double et de la seconde plaque de condensateur double, respectivement, ont une symétrie circulaire et sont disposées concentriquement l'une par rapport à l'autre.

9. Système selon l'une quelconque des revendications précédentes, dans lequel, dans la représentation des informations communiquées, un codage de Manchester est utilisé.

10. Système selon l'une quelconque des revendications précédentes, dans lequel les deux plaques de chacune des première et seconde plaques de condensateur double sont dotées de signaux qui sont l'inverse l'un de l'autre.

11. Système selon l'une quelconque des revendications précédentes, dans lequel chacune de la première et la seconde plaque de condensateur est connectée électriquement à un circuit d'attaque respectif, dans lequel les circuits d'attaque du premier et du second condensateur double ont un potentiel à la terre électrique commun.

12. Système selon la revendication 11, dans lequel le circuit d'attaque de l'unité de communication interne comprend ou est connecté à une partie électroconductrice pour être en contact électrique avec un tissu corporel interne lorsqu'il est implanté, et dans lequel le circuit d'attaque de l'unité de communication externe comprend ou est connecté à une partie électroconductrice conçue pour être électriquement connectée à la peau du corps de la personne.

13. Système selon la revendication 12, dans lequel la partie électroconductrice du circuit d'attaque de l'unité de communication interne fait partie d'un boîtier pour ledit circuit d'attaque.

14. Système selon la revendication 13, dans lequel la résistance électrique du trajet de communication est d'au moins 1 MΩ.

15. Système selon la revendication 1, dans lequel l'état ou les niveaux du signal comprennent au moins une parmi différentes fréquences du signal, différentes phases du signal et différentes caractéristiques d'impulsions telles que différents niveaux de tension et différents niveaux d'amplitude de l'onde porteuse.
